# EUROPÄISCHE PATENTSCHRIFT

(11) **EP 0 922 093 B1**
(45) Veröffentlichungstag und Bekanntmachung des Hinweises auf die Patenterteilung: **24.09.2003**
(21) Anmeldenummer: 97922797.2
(22) Anmeldetag: 02.06.1997
(51) Int. Cl.: C12N 5/06, C12N 5/08, C12M 3/06, A61L 27/00

(54) **VERFAHREN ZUR HERSTELLUNG VON KNORPELGEWEBE UND VON IMPLANTATEN**
METHOD FOR MAKING CARTILAGE AND IMPLANTS
PROCEDE DE PRODUCTION DE CARTILAGE ET D'IMPLANTS

(30) Priorität: 04.06.1996 CH 140896
(43) Veröffentlichungstag der Anmeldung: 16.06.1999
(73) Patentinhaber: Sulzer Orthopedics Ltd., 8404 Winterthur (CH)
(72) Erfinder: RIESER, Franz, CH-8542 Wiesendangen (CH); MÜLLER, Werner, CH-8542 Wiesendangen (CH); BITTMANN, Pedro, CH-8057 Zürich (CH); MAINIL-VARLET, Pierre, CH-3005 Bern (CH); SAAGER, Christoph, P., CH-3055 Frieswil (CH)
(74) Vertreter: Frei, Alexandra Sarah
(86) Internationale Anmeldenummer: CH9700220
(87) Internationale Veröffentlichungsnummer: WO97046665

(56) Entgegenhaltungen:
- WO-A-90/12603
- WO-A-93/19168
- DE-A- 4 306 661
- BIOMATERIALS, Bd. 17, Nr. 10, Mai 1996, GUILDFORD GB, Seiten 1049-1051, XP002024593 M. SITTINGER ET AL.: "ENCAPSULATION OF ARTIFICIAL TISSUES IN POLYELECTROLYTE COMPLEXES: PRELIMINARY STUDIES." in der Anmeldung erwähnt
- BIOMATERIALS, Bd. 15, Nr. 6, Mai 1994, GUILDFORD GB, Seiten 451-456, XP002024594 M. SITTINGER ET AL.: "ENGINEERING OF CARTILLAGE TISSUE USING BIORESORBABLE POLYMER CARRIERS IN PERFUSION CULTURE."
- CLINICAL ORTHOPAEDICS AND RELATED RESEARCH, Nr. 186, Juni 1984, LONDON, GB, Seiten 231-239, XP002024595 T. KIMURA ET AL.: "CHONDROCYTES EMBEDDED IN COLLAGEN GELS MAINTAIN CARTILAGE PHENOTYPE DURING LONG-TERM CULTURES."
- BIOMATERIALS, Bd. 10, Nr. 1, Januar 1989, Seiten 63-67, XP000604999 CHEUNG H S ET AL: "GORWTH OF OSTEOBLASTS ON POROUS CALCIUM PHOSPHATE CERAMIC: AN IN VITRO MODEL FOR BIOCOMPATILIGITY STUDY"

## Beschreibung

Die Erfindung liegt auf dem Gebiete der Medizinaltechnik und betrifft ein Verfahren gemäss dem Oberbegriff des ersten, unabhängigen Patentanspruchs zur Herstellung von Knorpelgewebe und von Implantaten zur Reparatur von enchondralen und osteochondralen Defekten. Ferner betrifft die Erfindung eine Anordnung zur Durchführung des Verfahrens und nach dem Verfahren hergestellte Implantate nach den Oberbegriffen der entsprechenden, unabhängigen Patentansprüche.

Knorpelgewebe besteht im wesentlichen aus Chondrozyten und extrazellulärer Matrix. Die extrazelluläre Matrix besteht hauptsächlich aus Kollagen Typ II und Proteoglycanen, deren Bausteine von den Chondrozyten in den zwischenzellulären Raum ausgeschieden werden, wo sie zu Makromolekülen zusammengebaut werden. Die Chondrozyten machen im Knorpelgewebe eines erwachsenen Individuums etwa 5% des Volumens aus.

Gelenkknorpel, der als knorpelige Schicht die Enden gelenkig verbundener Knochen überzieht, übernimmt die Funktion der Lastverteilung bei Belastung des Gelenkes. Für diese Funktion ist das Knorpelgewebe fähig, Wasser aufzunehmen und unter Druck wieder abzugeben. Ferner dienen die Gelenkknorpel-Oberflächen als Gleitflächen im Gelenk.

Knorpel ist ein Gewebe, das sich mangels Durchblutung insbesondere an erwachsenen Individuen kaum regeneriert, wenn das zu regenerierende Knorpelstück ein nur kleines Volumen überschreitet. Insbesondere Gelenkknorpel können aber oft altersbedingte Abnützungs- und Veränderungserscheinungen sowie unfallbedingte Verletzungen aufweisen, die ein derartiges maximal regenerierbares Volumen bei weitem überschreiten. Solche Beschädigungen der Knorpelschicht machen Bewegung und Belastung von betroffenen Gelenken schmerzhaft und führen zu weiteren Komplikationen wie beispielsweise Entzündungen bedingt durch Synovialflüssigkeit, die wegen des Defektes in der den Knochen abdeckenden Knorpelschicht mit dem Knochen in Kontakt kommt.

Aus diesen Gründen wird seit längerer Zeit versucht, fehlendes oder schadhaftes Knorpelgewebe, insbesondere Gelenkknorpel operativ zu ersetzen oder zu reparieren.

Es ist bekannt, Defekte, die Gelenkknorpel oder Gelenkknorpel und darunter liegendes Knochengewebe betreffen, zu reparieren, indem die defekte Stelle zu einer Bohrung mit einer möglichst genau definierten Geometrie ausgebohrt wird, indem an einer nicht defekten, vorzugsweise wenig belasteten Stelle beispielsweise desselben Gelenkes eine Säule bestehend aus Knorpel und darunterliegendem Knochen mit möglichst derselben Geometrie, wie die Bohrung sie hat, ausgestanzt/ausgebohrt wird und indem die ausgestanzte Säule in die Bohrung eingesetzt wird. In derselben Weise werden auch grössere Defekte mit einer Mehrzahl von Säulen repariert (Mosaikplastik). Diese Methoden sind erfolgreich aber das eigentliche Problem wird im wesentlichen von einer belasteten Gelenkstelle an eine unbelastete Gelenkstelle verschoben und nicht effektiv gelöst.

Es wird auch vorgeschlagen, beispielsweise in der Publikation US-3703575 (Thiele), Knorpeldefekte mit rein künstlichen Implantaten (z.B. Gele, die Proteine und Polysaccharide enthalten) zu reparieren. Es zeigt sich aber, dass damit nur beschränkte Erfolge erzielt werden können, weshalb in der neueren Entwicklung andere Wege beschritten werden, die alle von autologen oder homologen, vitalen Zellen ausgehen. Es werden beispielsweise in vitro gezüchtete, vitale Chondrozyten oder Zellen, die eine Chondrozytenfunktion ausüben können, implantiert; oder es werden künstliche Implantate benützt, in die vitale Chondrozyten eingebracht sind; oder es wird versucht, in vitro Knorpelgewebe zu züchten und dieses zu implantieren. Das heisst mit anderen Worten, nach neuerer Entwicklung wird versucht, vitales Knorpelgewebe mindestens teilweise in vitro zu erzeugen und zu implantieren oder knorpelbildende Zellen im zu reparierenden Defekt anzusiedeln, welche Zellen in vivo ein mindestens Knorpel-ähnliches Gewebe bilden.

Beispiele derartiger Verfahren sind in den folgenden Publikationen beschrieben:

Nach dem Verfahren gemäss US-4846835 (Grande) werden körpereigene Chondrozyten nach einer vorgängigen Vermehrung in einer Monolayer-Kultur für eine weitere Vermehrung in eine dreidimensionale Kollagenmatrix in Form eines Gels oder eines Schwammes eingebracht, in welcher Matrix sie sich festsetzen und dadurch immobilisiert sind. Nach ca. drei Wochen Zellvermehrung wird die defekte Knorpelstelle mit dem Material bestehend aus Kollagenmatrix und Zellen gefüllt und, um das Transplantat an der defekten Stelle festzuhalten, wird ein Stück Knochenhaut darüber genäht. Die Knorpelregeneration im Bereiche derartiger Transplantate ist bedeutend besser als ohne Transplantat.

Nach dem Verfahren gemäss US-5053050 (Itay) werden Chondrozyten oder Zellen, die eine Chondrozyten-Funktion ausüben können, in eine biokompatible, resorbierbare Matrix eingebracht (32x10⁶ bis 120x10⁶ Zellen pro cm³), in welcher Matrix die Zellen immobilisiert sind. Diese Matrix wird implantiert, wobei sich in vivo ein knorpelähnliches Gewebe bildet. Die für das Implantat verwendeten Chondrozyten werden vorgängig kultiviert und zwar zuerst in einer Monolayer-Kultur und dann suspendiert, wobei sie sich zu Aggregaten von 30 bis 60 Zellen verbinden.

Nach dem Verfahren gemäss US-4963489 (Naughton) wird ebenfalls eine dreidimensionale künstliche Matrix als Trägermaterial für cas Implantat verwendet. Diese wird bereits für die Zellkultur verwendet und ist zur besseren Adhäsion und Versorgung der zu kultivierenden Zellen mit einer vitalen Bindegewebeschicht überzogen. Nach einer in vitro durchgeführten Zellvermehrung auf der dreidimensionalen Matrix, wird diese implantiert. Die implantierten Zellen bilden in vivo das Knorpelgewebe.

Nach dem Verfahren gemäss PCT-W090/12603 (Vacanti et al.) wird ebenfalls eine dreidimensionale Matrix verwendet, die aus abbaubaren, polymeren Fasermaterialien besteht und an die sich Zellen anlagern lassen. Die Zellen werden in vitro bereits auf der Matrix oder in Monolayer-Zellkulturen vermehrt und die Matrix wird mit den an ihr haftenden und dadurch immobilisierten Zellen implantiert. Die Matrix wird in vivo abgebaut und sukzessive durch extrazelluläre Matrix, die von den Zellen gebildet wird, ersetzt.

Nach dem Verfahren gemäss US-5326357 (Kandel) werden Chondrozyten auf einer Schicht aus Filtermaterial (MILICELL®-CM mit einer Porengrösse von 0,4µm) als Monolayer mit einer Dichte von 1,5x10⁶ Zellen pro cm² aufgebracht. Eine in vitro Kultur des Monolayers auf dem Filtermaterial ergibt in zwei bis vier Wochen eine dünne Knorpelschicht, die in ihrem Aufbau offenbar dem natürlichen Gelenkknorpel entspricht, und die als solche implantierbar ist.

Es ist auch bekannt, dass Knorpel in sogenannten high density Zellkulturen gezüchtet werden kann. Dabei werden Zellen in einer höheren Dichte als für einen Monolayer notwendig auf einen Träger gebracht und kultiviert. Erst nach ein bis zwei Stunden wird das Kulturmedium zugegeben. Nach zwei beis drei Tagen kontrahiert sich die Zellschicht auf dem Träger und bilden sich sogenannte Microspheres mit Durchmessern in der Grössenordnung von 1mm. Im Innern dieser Microspheres bildet sich in der anschliessenden Kultur ein Knorpel-artiges Gewebe, während an deren Oberfläche Faserknorpel (Perichondrium) entsteht. Für Implantate ist ein derartig inhomogenes Gewebe wenig geeignet.

Sittinger er al. (Biomaterials Bd. 17, Nr. 10, Mai 1996, Guildford GB) schlagen auch vor, für die Bildung von Knorpel in vitro in eine dreidimensionale Matrix vitale Zellen einzubringen und die beladene Matrix dann in eine semipermeable Membran einzuschliessen. Durch die Membran wird vor allem verhindert, dass während der Knorpelbildung von den Zellen für den Aufbau einer extrazellulären Matrix ausgeschiedene Bauteile mit dem Kulturmedium ausgeschwemmt werden. Es ist auch bekannt, Zellkulturen zur Verhinderung von Immunabwehrreaktionen in eine derartige Membran eingeschlossen zu implantieren.

Alle oben genannten Verfahren, mit denen versucht wird, Knorpel mindestens teilweise in vitro herzustellen, stellen verschiedene Lösungen eines länger bekannten Problems dar, das sich der Herstellung von Knorpelgewebe durch natürliche Zellen aber unter künstlichen Bedingungen stellt. Dieses Problem besteht darin, dass Chondrozyten unter derartigen in vitro Bedingungen die Tendenz haben, relativ schnell zu Fibroblasten zu dedifferenzieren, bzw. dass es nur unter sehr spezifischen Kulturbedingungen möglich ist, Fibroblasten zu einer Chondrozyten-Funktion zu differenzieren. Durch die Dedifferenzierung verlieren Chondrozyten unter anderem ihre Fähigkeit zur Bildung von Typ II Collagen, das einen wichtigen Bestandteil von Knorpelgewebe darstellt.

Das Problem der Dedifferenzierung der Chondrozyten wird gemäss den oben genannten Verfahren dadurch gelöst, dass die Chondrozyten in entsprechend dichten Kulturen in einem Monolayer oder in einer dreidimensionalen Matrix immobilisiert werden. Es zeigt sich, dass Chondrozyten sich auf diese Art ohne wesentliche Dedifferenzierung vermehren und extrazelluläre Matrix bilden, die der extrazellulären Matrix von natürlichem Knorpel mindestens ähnlich ist. Die dreidimensionale Matrix dient in den meisten Fällen nicht nur zur Immobilisierung der Zellen sondern auch als belastbarer Körper nach der Implantation, denn die gebildeten Knorpelgewebe haben in keinem bekannten Fall eine Stabilität, die nach der Implantation einer auch nur reduzierten Belastung standhalten könnte.

Die Erfindung stellt sich die Aufgabe, ein Verfahren zu schaffen, mit dem in einfacher Weise für Implantationen, insbesondere für Implantationen bei Gelenkknorpel-Defekten. geeignetes Knorpelgewebe in vitro herstellbar wird oder Implantate herstellbar werden, die mindestens zum Teil aus derartigem Knorpelgewebe bestehen. Zur Lösung dieser Aufgabe ist es notwendig, für Chondrozyten bzw. andere, zu einer Chondrozytenfunktion fähige Zellen in vitro eine derartige Umgebung, insbesondere eine dreidimensionale derartige Umgebung zu schaffen, dass sie auch über eine längere Kulturzeit nicht dedifferenzieren und ihre Funktion aktiv ausüben, bzw. dass sie zu aktiven Chondrozyten differenziert werden. Durch die Lösung dieses Problems ist der Hauptteil der Aufgabe gelöst, denn nicht dedifferenzierende, vitale Chondrozyten bauen unter den geeigneten Bedingungen gemäss ihrer natürlichen Funktion die entsprechende extrazelluläre Matrix auf und stellen zusammen mit dieser Knorpelgewebe dar.

Die nach dem erfindungsgemässen Verfahren hergestellten Implantate, die mindestens zum Teil aus in vitro hergestelltem Knorpelgewebe bestehen, sollen insbesondere geeignet sein für die Reparatur von enchondralen oder osteochondralen Gelenk-Defekten. Sie sollen für jede Tiefe derartiger Defekte herstellbar sein und mit den erfindungsgemässen Implantaten reparierte derartige Defekte sollen möglichst bald nach der Implantation belastbar sein und zwar sowohl mit Press- als auch mit Scherkräften.

Die oben genannte Aufgabe wird gelöst durch das Verfahren, wie es in den Ansprüchen definiert ist.

Das erfindungsgemässe Verfahren beruht auf dem Befund, dass Chondrozyten ein den Ansprüchen genügendes Knorpelgewebe aufbauen können, wenn dafür gesorgt wird, dass die Konzentration von Stoffen, die von den Zellen produziert und in extrazelluläre Räume ausgeschieden werden, nach einer kurzen Anfangsphase eine genügend hohe Konzentration aufweisen und dass eine ähnliche Konzentration während der ganzen Kulturzeit aufrechterhalten bleibt. Unter diesen Bedingungen bleibt die differenzierte Funktion von Chondrozyten voll erhalten (sie dedifferenzieren nicht zu Fibroblasten) und/oder ist es möglich, entsprechende Zellen, insbesondere mesenchymale Stammzellen oder andere mesenchymale Zellen oder auch Fibroblasten zu einer entsprechenden Funktion zu differenzieren.

Die erste Bedingung wird erfüllt dadurch, dass eine Zellgemeinschaft geschaffen wird, in der mindestens zu Beginn der Kultur eine derart hohe Zelldichte herrscht, dass die Zellen fähig sind, die für die genannten Konzentrationen in den Zellzwischenräumen notwendigen Mengen an Stoffen in der genannten kurzen Zeit zu produzieren. Die zweite Bedingung wird erfüllt dadurch, dass die Zellgemeinschaft in einem begrenzten Zellraum untergebracht wird, in dem verhindert wird, dass die genannten Stoffe aus den Zellzwischenräumen ausgeschwemmt werden.

Die genannten Stoffe sind insbesondere autokrine Faktoren und Stoffe, die als Bausteine für den Aufbau der extrazellulären Matrix dienen. Diese Bausteine sind vor allem Aggrekane, Link-Proteine und Hyalorunate für den Aufbau der Proteoglykan-Aggregate und Kollagen-Vorstufen für den Aufbau der Kollagenfibrillen vom Typ II.

Gemäss dem erfindungsgemässen Verfahren werden die Zellen für die in vitro Kultur nicht immobilisiert sondern es wird ihnen ein Raum ohne dreidimensionale, künstliche Matrix zur Verfügung gestellt, in dem die beiden oben angegebenen Bedingungen erfüllt sind, in dem es aber weitgehend den Zellen überlassen wird, wie sie sich relativ zueinander ansiedeln wollen. Es zeigt sich, dass in einem derartigen freien Zellraum Zellen ihre Chondrozytenfunktion voll ausüben und sich ein Knorpelgewebe züchten lässt, das eine für eine Implantation und für eine mindestens beschränkte Belastung nach der Implantation genügende Festigkeit aufweist.

Nach dem erfindungsgemässen Verfahren werden also Zellen, die zu einer Chondrozytenfunktion befähigt sind, in einen an sich leeren, das heisst höchstens mit Kulturmedium gefüllten Zellraum eingebracht derart, dass im Zellraum eine Zelldichte von etwa 5x10⁷ bis 10⁹ Zellen pro cm³ Zellraum entsteht, was bei einem ungefähren Zellvolumen jeder Zelle von 10³ µm³ eine Raumbelegung von ca. 5% bis 100% ergibt.

Der Zellraum hat mindestens teilweise durchlässige Wände und wird für die Kulturzeit in einen mit Kulturmedium gefüllten Raum eingebracht, welches Kulturmedium in bekannter Weise periodisch erneuert wird. Während der Kulturzeit ist der Zellraum stationär im Kulturmedium angeordnet oder wird darin bewegt (relative Bewegung zwischen Zellraum und den Zellraum umgebendem Kulturmedium).

Die Durchlässigkeit der durchlässigen Teile der Zellraumwandung und die relative Bewegung von Zeitraum und Kulturmedium, sind derart auf die Dimensionen des Zellraumes (abhängig vom zu erzeugenden Knorpel) abzustimmen, dass die Bedingung der Ausschwemmungs-Verhinderung erfüllt wird.

Für alle Fälle sind semipermeable Wandbereiche (semipermeable Membrane) mit einer Durchlässigkeit von 10'000 bis 100'000 Dalton (10 bis 100 kDa) geeignet, insbesondere für bewegte Kulturen und für Zellräume mit grossen Dimensionen (dreidimensionale Gebilde). Die genannten autokrinen Faktoren und Bausteine für den Aufbau der Makromoleküle der extrazellulären Knorpelmatrix haben Molekulargewichte derart, dass sie eine Membran mit der genannten Duchlässigkeit nicht passieren können.

Es zeigt sich, dass für stationäre Kulturen und Zellräume mit mindestens einer kleinen Dimension (dünne Schichten) auch offenporige Wände mit bedeutend grösseren Poren (bis in den Bereich von 10 bis 20µm), die nicht als semipermeable Wände sondern nur als Konvektionsbarrieren wirken können, genügen und dass gegebenenfalls der Zellraum sogar auf einer Seite offen sein kann.

Insbesondere in unbewegten Zellräumen setzen sich die Zellen bei kleineren Zelldichten in Richtung Schwerkraft ab, wodurch schichtartige Knorpelgewebe erzeugbar sind. Für die Erzeugung mehr dreidimensionaler Knorpelgebilde erweisen sich bewegte Zellräume als vorteilhaft.

Für spezifische Kulturen und insbesondere auch für spezifische Formen und Grössen von Zellräumen muss die optimale Anordnung (mit oder ohne Bewegung des Zellraumes im Kulturmedium) und die optimale Beschaffenheit der Zellraumwand bzw. der durchlässigen Teile der Zellraumwand experimentell ermittelt werden.

Der Zellraum hat also im wesentlichen drei Funktionen:
- Der Zellraum hält die Gemeinschaft der (nicht immobilisierten) Zellen in einer genügenden Dichte zusammen, derart, dass sie ihre spezielle Funktionsfähigkeit nicht verlieren;
- der Zellraum begrenzt das Knorpelwachstum, sodass durch Wahl der Zellraumform die Form des entstehenden Knorpels steuerbar ist;
- die Zellraumwand erlaubt die Versorgung der Zellen mit Kulturmedium, verhindert aber die Ausschwemmung der für das Knorpelwachstum notwendigen, von den Zellen sekretierten Stoffe.

Für die Herstellung von Implantaten, die nur teilweise aus Knorpelgewebe bestehen, kann ein Teil der durchlässigen Zellraumwand zusätzlich noch eine Funktion als Implantatsteil übernehmen, wie weiter unten noch im Detail zu beschreiben sein wird.

Die in den Zellraum einzubringenden Zellen sind Chondrozyten. mesenchymale Stammzellen oder andere mesenchymale Zellen. Diese Zelltypen können aus Knorpelgeweben, Knochen oder Knochenmark in bekannter Weise isoliert werden oder auch aus Binde- oder Fettgewebe. Auch Fibroblasten sind geeignet, wenn dem Kulturmedium bzw. dem Zellraum Faktoren zugegeben werden, die ihre Differenzierung zu Chondrozyten bewirken oder die Zellen, bevor sie in den Zellraum gebracht werden, mit derartigen Faktoren behandelt werden. Die Zellen können auch, bevor sie in den Zellraum eingebracht werden, in vitro vermehrt werden.

Die in den genannten Geweben vorhandenen Mischungen von Zelltypen brauchen nicht aufgetrennt zu werden, sondern können als solche in den Zellraum eingebracht werden. Es zeigt sich auch, dass eine vollständige Trennung der Zellen von der sie umgebenden interzellulären Matrix nicht notwendig ist, dass also gegebenenfalls anstelle von isolierten Zellen auch Gewebeteilchen oder Mischungen von isolierten Zellen und Gewebeteilchen in den Zellraum eingebracht werden können. Dabei ist gegebenenfalls durch teilweise Abtrennung der extrazellulären Matrix von solchen Gewebeteilchen, beispielsweise durch eine kurze enzymatische Verdauung, dafür zu sorgen, dass die geforderte Zelldichte im Zellraum erreicht werden kann.

Es zeigt sich, dass mit handelsüblichen Kulturmedien, wie beispielsweise HAM-F12, denen vorteilhafterweise 5 bis 15% Serum zugegeben werden, gute Resultate erzielt werden können. Ferner können dem Kulturmedium auch an sich bekannte Wachstumsfaktoren und andere Komponenten von Kulturmedien, die eine Vermehrung der Zellen und die Bildung der Knorpelmatrix fördern, beigegeben werden.

Es zeigt sich, dass in den gemäss dem erfindungsgemässen Verfahren erzeugten Kulturbedingungen die Chondrozyten aktiv bleiben und nicht dedifferenzieren, sodass sich der Raum in einer Kulturzeit in der Grössenordnung von ca. drei Wochen mit Knorpelgewebe füllt. Das entstehende Knorpelgewebe kann, sobald es dazu eine genügende mechanische Festigkeit aufweist, aus dem Zellraum entfernt werden und beispielsweise frei schwimmend in einem Kulturmedium weiter kultiviert werden oder es kann bis unmittelbar vor der Weiterverwendung (Transplantation) im Zellraum verbleiben.

Nach dem erfindungsgemässen Verfahren hergestelltes Knorpelgewebe kann als Implantat oder Implantatsteil, bzw. bei der Verwendung von autologen Zellen als Zell-auto-Transplantat oder aber auch für wissenschaftliche Zwecke in vitro weiterverwendet wird.

Die folgenden Figuren illustrieren das erfindungsgemässe Verfahren, die Anordnung zur Durchführung des erfindungsgemässen Verfahrens sowie Beispiele von Implantaten, die durch das erfindungsgemässe Verfahren hergestellt werden können. Bei den dargestellten Beispielen handelt es sich um Implantate für die Reparatur von enchondralen und osteochondralen Defekten. Mit dem erfindungsgemässen Verfahren ist es aber auch möglich, andere Implantate herzustellen, zum Beispiel Gehörknochen oder für die plastische Chirurgie Nasenknorpel, Orbitalböden, Ohrenmuscheln oder Teile davon.
**Figuren 1 bis 4** zeigen vier beispielhafte Anordnungen (im Schnitt) zur Durchführung des erfindungsgemässen Verfahrens zur in vitro Herstellung von Knorpelgewebe;
**Figuren 5 bis 7** zeigen Chondroitin- und Kollagengehalt von verschiedenen experimentellen Knorpelkulturen (aus Kulturen nach dem erfindungsgemässen Verfahren und aus Vergleichskulturen nach bekannten Verfahren) im Vergleich mit Knorpel aus einem Gelenk;
**Figuren 8 und 9** zeigen zur Illustration der mechanischen Eigenschaften von nach dem erfindungsgemässen Verfahren hergestelltem Knorpel: Kraftverläufe bei der Belastung dieses Knorpels (Figur 8) und bei der Belastung eines nativen Knorpels (Figur 9);
**Figuren 10 bis 13** zeigen licht- und elektronenmikroskopische Aufnahmen von nach dem erfindungsgemässen Verfahren hergestelltem Knorpel und von nativem Knorpel.
**Figur 14** zeigt einen Schnitt durch ein nach dem erfindungsgemässen Verfahren gemäss Figur 2 oder 3 hergestelltes Implantat mit einer Trägerschicht und einer Knorpelschicht (Grenzbereich zwischen den zwei Schichten im Schnitt senkrecht zu den Schichten);
**Figuren 15 und 16** zeigen beispielhafte Ausführungsformen des erfindungsgemässen Implantates gemäss Figur 14 im Schnitt senkrecht zur Knorpelschicht;
**Figuren 17 bis 20** zeigen Beispiele von Anwendungen von nach dem erfindungsgemässen Verfahren hergestellten Implantaten zur Reparatur von enchondralen und osteochondralen Gelenk-Defekten (Schnitte senkrecht zur Knorpelschicht).

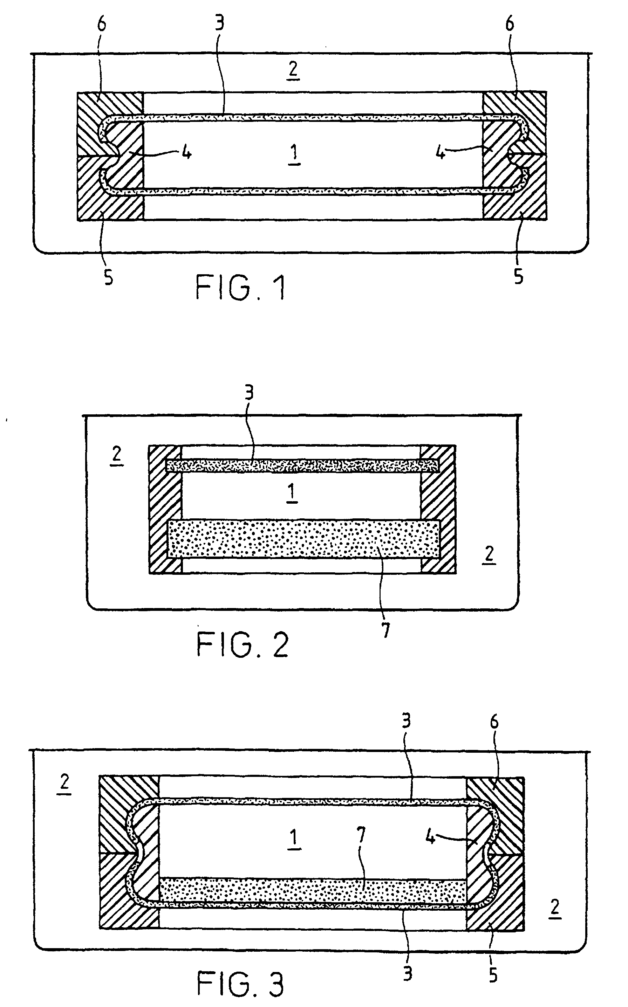

**Figur 1** zeigt im Schnitt eine beispielhafte Anordnung zur erfindungsgemässen in-vitro-Herstellung von Knorpelgewebe. Diese besteht im wesentlichen aus einem begrenzten Zellraum 1, in den die Zellen eingebracht werden und der in einem Kulturmedium-Raum 2 angeordnet ist. Mindestens ein Teil der Begrenzung des Zellraumes 1 gegen den Kulturmedium-Raum 2 wird durch eine durchlässige Wand, beispielsweise eine semipermeable Membran 3 gebildet. Die restliche Begrenzung des Zellraumes 1 gegen den Kulturmedium-Raum 2 ist dicht und besteht beispielsweise aus Kunststoffteilen, die dem Zellraum 1 die vorgesehene Form verleihen und die semipermeablen Membrane festhalten.

Im dargestellten Beispiel sind ein innerer Ring 4 und zwei auf diesen inneren Ring 4 aufsteckbare äussere Schnappringe 5 und 6 vorgesehen, die zusammen mit zwei beispielsweise im wesentlichen kreisförmigen Stücken semipermeabler Membran 3 einen kreisscheibenförmigen Zellraum 1 einschliessen.

Die semipermeable Membran 3 hat eine Durchlässigkeit von 10'000 bis 100'000 Dalton. Sie besteht zum Beispiel aus demselben Material wie ein entsprechender Dialyseschlauch.

Es ist offensichtlich, dass in analoger Art zu der in der Figur 1 dargestellten Anordnung Zellräume verschiedenster Form gebildet werden können, in die Zellen eingebracht werden und in denen diese Knorpelgewebe aufbauen, wobei das Knorpelgewebe im wesentlichen die Form des Zellraumes 1 oder des während der Kultur in Richtung Schwerkraft gegen unten gerichteten Teils des Zellraumes 1 annimmt.

Der Kulturmedium-Raum 2 ist ein beliebiger Raum, in dem in bekannter Weise das Kulturmedium periodisch ausgewechselt wird. Wenn der Zellraum 1 im Kulturmedium-Raum 2 bewegt werden soll, ist der Kulturmedium-Raum beispielsweise eine Spinnerflasche.

Unter Verwendung einer Anordnung gemäss Figur 1 läuft das erfindungsgemässe Verfahren beispielsweise wie folgt ab:
- Zellen, Gewebeteilchen oder Mischungen von Zellen und/oder Gewebeteilchen, wie sie weiter oben beschrieben sind, werden, z.B. in Kulturmedium aufgeschlämmt, in den freien Zellraum eingebracht derart, dass die Zelldichte im Zellraum im Bereiche zwischen 5x10⁷ und 10⁹ Zellen pro cm³ beträgt.
- Der Zellraum wird geschlossen, dann in den Kulturmedium-Raum eingebracht und darin für einen Zeitraum in der Grösse von etwa drei Wochen belassen.
- Das im Zellraum entstandene Knorpelgewebe wird aus dem Zellraum entfernt und entweder in einem Kulturmedium frei schwimmend weiter kultiviert oder direkt als Implantat bzw. Transplantat oder für wissenschaftliche Untersuchungen weiterverwendet.

In Zellräumen gemäss Figur 1 werden Implantate hergestellt, die nur aus Knorpelgewebe bestehen, beispielsweise Gehörknochen, Nasenknorpel, Orbitalböden Ohrenmuscheln oder Teile davon.

**Figur 2** zeigt eine weitere Variante eines Zellraumes für die Durchführung des erfindungsgemässen Verfahrens. Der Zellraum 1 ist flach und seine eine Seite ist begrenzt durch eine offenporige, starre oder plastisch deformierbare Platte 7 aus einem biologisch abbaubaren oder nicht abbaubaren Knochenersatzmaterial, die andere Seite durch eine weitere durchlässige Wand, beispielsweise eine semipermeable Membran 3 oder eine gröber poröse Wand. Der Zellraum hat eine Höhe von beispielsweise ca. 3 bis 5mm und eine beliebige flächige Form und Ausdehnung.

Ein Zellraum, wie er in der Figur 2 dargestellt ist, ist insbesondere geeignet für die Herstellung eines Implantats zur Reparatur eines osteochondralen Defekts, welches Implantat nicht nur die in dem flachen Zellraum gewachsene Knorpelschicht sondern auch die Knochenersatzplatte 7 umfasst. Diese Knochenersatzplatte 7 hat also im wesentlichen zwei Funktionen: sie dient während dem Knorpelwachstum als durchlässige Wandung des Zellraumes 1 und sie dient im fertigen Implantat als Verankerungssubstrat für die Knorpelschicht. wobei nach der Implantation dieses Knochenersatzmaterial in bekannter Weise von Zellen aus dem angrenzenden, vitalen Knochen besiedelt wird.

Damit die Knochenersatzplatte 7 die oben genannte zweite Funktion erfüllen kann, muss durch entsprechende Anordnung des Zellraumes im Kulturmedium-Raum dafür gesorgt werden, dass die Zellen sich möglichst regelmässig auf der Knochenersatzplatte 7 absetzen (mindestens für den Fall, dass eine Zelldichte gewählt wird, die zu einem Absetzen der Zellen führt). Der Zellraum 1 wird also beispielsweise, wie in der Figur 2 dargestellt, mit der Knochenersatzplatte 7 gegen unten stationär im Kulturmedium-Raum 2 angeordnet, sodass die Zellen sich durch die Wirkung der Schwerkraft auf der Knochenersatzplatte 7 absetzen.

Ferner muss die Knochenersatzplatte 7 derart beschaffen sein, dass das im Zellraum 1 entstehende Knorpelgewebe in einem Grenzbereich mit der Knochenersatzplatte 7 verwächst und dadurch ein auf Scherung belastbares Implantat entsteht. Eine derartige Verwachsung wird erreicht dadurch, dass die Porosität des Knochenersatzmaterials mindestens auf derjenigen Oberfläche, auf der der Knorpel gezüchtet wird, so gewählt wird, dass die bei der Bildung der extrazellulären Knorpelmatrix entstehenden Kollagenfibrillen in die Poren hineinwachsen und den entstehenden Knorpel in der Knochenersatzplatte verankern. Es zeigt sich, dass für eine derartige Verankerung der Kollagenfibrillen Poren von ca. 1 bis 20µm geeignet sind.

Ferner ist es vorteilhaft, wenn von den Zellen, die für die Knorpelzüchtung auf die Oberfläche der Knochenersatzplatte aufgebracht werden sich ein Teil in Oberflächenunebenheiten oder Poren niederlässt, sodass durch das Wachsen von Knorpelgewebe in diesen Unebenheiten oder Poren Knorpelgewebe und Knochenersatzmaterial in der Art eines Formschlusses miteinander verbunden werden. Es zeigt sich, dass Zellen sich leicht in Unebenheiten oder Poren niederlassen, wenn diese mindestens 20µm, vorteilhafterweise zwischen 20 und 50µm gross sind.

An die Knochenersatzplatte 7 werden also die folgenden Bedingungen gestellt:
- Damit die Zellen sich aus dem Kulturmedium durch die Knochenersatzplatte ernähren können, muss diese Poren aufweisen, die durchgehende Kanäle bilden (offene Porosität).
- Damit die Knochenersatzplatte mindestens als Konvektionsbarriere gegen die Ausschwemmung der grösseren Moleküle dienen kann, dürfen die Poren nicht zu gross und die Plattendicke nicht zu klein sein.
- Damit die beim Knorpelwachstum entstehenden Kollagenfibrillen sich in den Poren der Platte verankern, sollen die Poren nicht grösser als ca. 20µm sein.
- Damit Zellen sich in Unebenheiten der Oberfläche der Knorpelersatzplatte ansiedeln können, müssen mindestens auf der gegen die wachsende Knorpelschicht gewandten Oberfläche der Knochenersatzplatte derartige Unebenheiten (Oberflächenrauheit) mit Grössen von mindestens ca. 20µm vorgesehen sein.

Es zeigt sich dass mit entsprechend Oberflächen-rauhen Knochenersatzplatten mit offenen Poren in der Grössenordnung von 2 bis 20µm und mit einer Dicke von 0,5 bis 3mm, vorzugsweise 0,5 bis 1,5mm gute Resultate erzielt werden können. Bei Plattendicken über ca. 0,5 bis 1mm kann der von der wachsenden Knorpelschicht abgewandte Bereich der Knochenersatzplatte auch eine gröbere Porosität aufweisen, beispielsweise Poren bis zu Grössen von 300 bis 700µm, wie sie von Knochenersatzmaterialien bekannt sind. Derartige Porositäten begünstigen die in vivo Vaskularisierung des Knochenersatzmaterials.

Als Knochenersatzmaterial eignen sich an sich alle gängigen osteoinduktiven und/oder osteokonduktiven Materialien, vorzugsweise biologisch abbaubare Materialien, die die angegebene, offene Porosität aufweisen und die sich zu starren oder plastisch verformbaren Platten verarbeiten lassen. Beispielsweise sind plastisch verformbare Platten herstellbar aus Kollagen I, aus Kollagen I und Hydroxyapatit oder aus Polymilchsäure, starre Platten aus Tricalziumphosphat, aus Hydroxyapatit und aus anderen anorganischen Knochenersatzmaterialien.

Eine Behandlung der Knochenersatzplatte 7 mit einem Haftfaktor (attachment factor) ist nicht notwendig.

In einem Zellraum gemäss Figur 2 mit einer entsprechend offenporigen Knochenersatzplatte 7 wird also nicht nur Knorpelgewebe in vitro gezüchtet, sondern es wird ein Implantat hergestellt, das einen vorgebildeten, verwachsenen Knorpel/Knochen-Grenzbereich aufweist.

**Figur 3** zeigt eine weitere, beispielhafte Anordnung zur Durchführung des erfindungsgemässen Verfahrens. Es handelt sich im Prinzip um eine Kombination des Verfahrens gemäss Figuren 1 und 2. Die Knochenersatzplatte 7 ist hier nicht als Teil der durchlässigen Wandung des Zellraumes 1 angeordnet sondern liegt innerhalb eines derartigen, beispielsweise mit semipermeablen Membranen 3 begrenzten Raumes. Es ist offensichtlich, dass in einer derartigen Anordnung die Funktion der Knochenersatzplatte 7 als Konvektionsbarriere wegfällt und dass aus diesem Grunde die Porosität und Dicke der Platte mit einer grösseren Freiheit gewählt werden kann.

Die in der Figur 3 dargestellte Anordung eignet sich insbesondere für dünne, plastisch verformbare Knochenersatzplatten 7, die als Zellraumwandung schwierig zu handhaben sind.

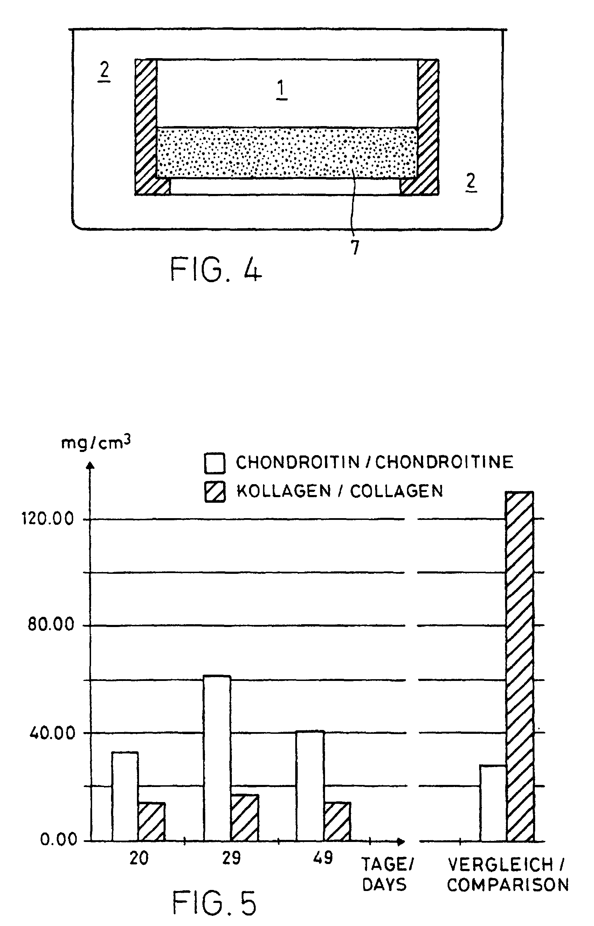

**Figur 4** zeigt schematisch eine weitere Ausführungsform eines Zellraumes 1, der sich insbesondere zur Züchtung von sehr dünnen Knorpelschichten, die mit einer Knochenersatzplatte verwachsen sind, eignet. Im Gegensatz zum Zellraum gemäss Figuren 1 bis 3 ist der Zellraum der Figur 4 gegen oben offen, sodass mindestens in den ersten ein bis zwei Wochen unter stationären Kulturbedingungen gezüchtet werden muss. Im Gegensatz zu ähnlichen, bekannten Anordnungen (z.B. US-5326357, Kandel) ist als Träger für die Zellen eine Knochenersatzplatte 7 vorgesehen und werden die Zellen nicht als Monolayer auf den Träger aufgebracht und nicht mit Hilfe eines Haftfaktors immobilisiert.

**Figuren 5 bis 7** zeigen Resultate, die in Versuchen mit dem erfindungsgemässen Verfahren (Versuchsanordnung im wesentlichen wie in Figur 1 skizziert) erhalten wurden. Als semipermeable Membran wurden Dialyseschläuche verwendet.

Es wurden aus Schultergelenken von Rindern isolierte Chondrozyten verwendet. Die Isolierung der Zellen wurde nach bekannten Methoden durchgeführt. Die Zellen wurden in die Dialyseschläuche eingebracht und diese während der Kulturzeit in einer Spinnerflasche bewegt, wobei die Zellen sich am unteren Ende der Schläuche absetzten. Als Kulturmedium wurde HAM-F12 mit 5 bis 15% Serum verwendet. Das Kulturmedium wurde alle zwei Tage gewechselt.

**Figur 5** zeigt die Gehalte an Chondroitinsulfat und Kollagen (µg/ml, auf das Volumen des gebildeten Knorpelgewebes bezogen) als Funktion der Kulturzeit (20, 29 und 49 Tage) des nach dem erfindungsgemässen Verfahren hergestellten Knorpelgewebes verglichen mit entsprechenden Werten von Knorpel aus dem Schultergelenk eines achtzehn Monate alten Rindes (Vergleich). Die Resultate zeigen, dass der Gehalt an Chondroitinsulfat im in vitro hergestellten Knorpelgewebe sogar höher sein kann als in natürlichem Knorpel, dass aber der Gehalt an Kollagen deutlich tiefer ist.

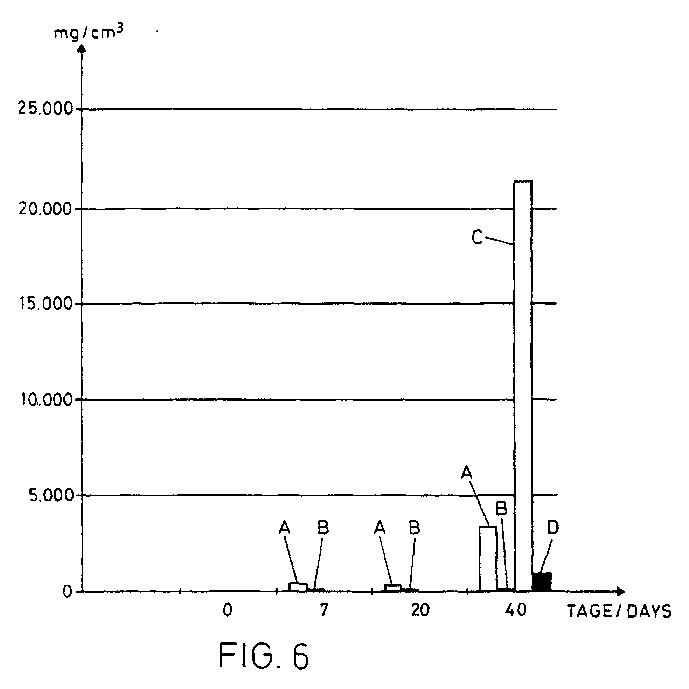

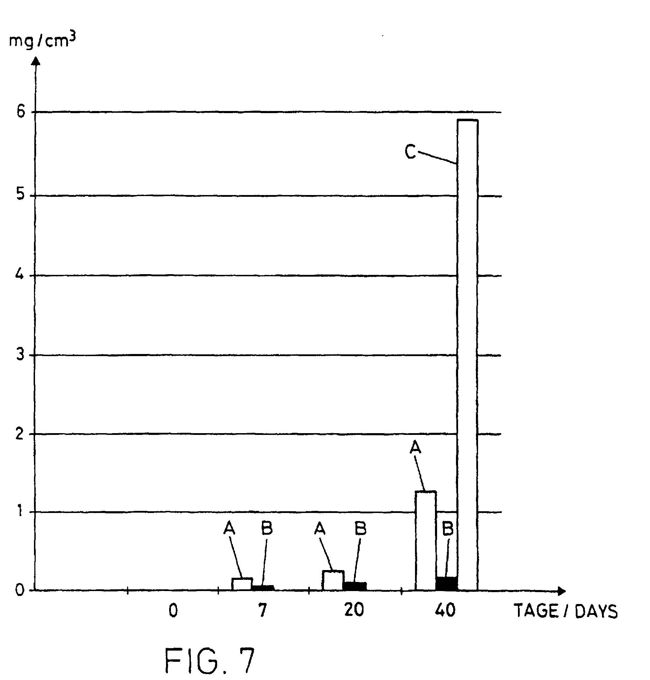

**Figuren 6 und 7** zeigen ebenfalls als Funktion der Kulturzeit (0, 7, 20 und 40 Tage) Chondroitinsulfat- (Figur 6) und Kollagen-Gehalte (Figur 7) in Vergleichskulturen A und B und von nach dem erfindungsgemässen Verfahren gezüchtetm Knorpelgewebe nach 40 Tagen Kulturzeit (C: entstehender Knorpel im Bereich der abgesetzten Zellen im Zellraum, D: Kulturmedium über den abgesetzten Zellen im Zellraum). Als Vergleichsversuche wurden Chondrozyten in Alginatkugeln eingebettet und in einer stationären Kultur kultiviert (A) und in einer Spinnerflasche (B).

Durch die Figuren 6 und 7 wird deutlich, dass der Knorpelaufbau in der Versuchsanordnung gemäss Erfindung bedeutend erfolgreicher verläuft als in den Vergleichsversuchen.

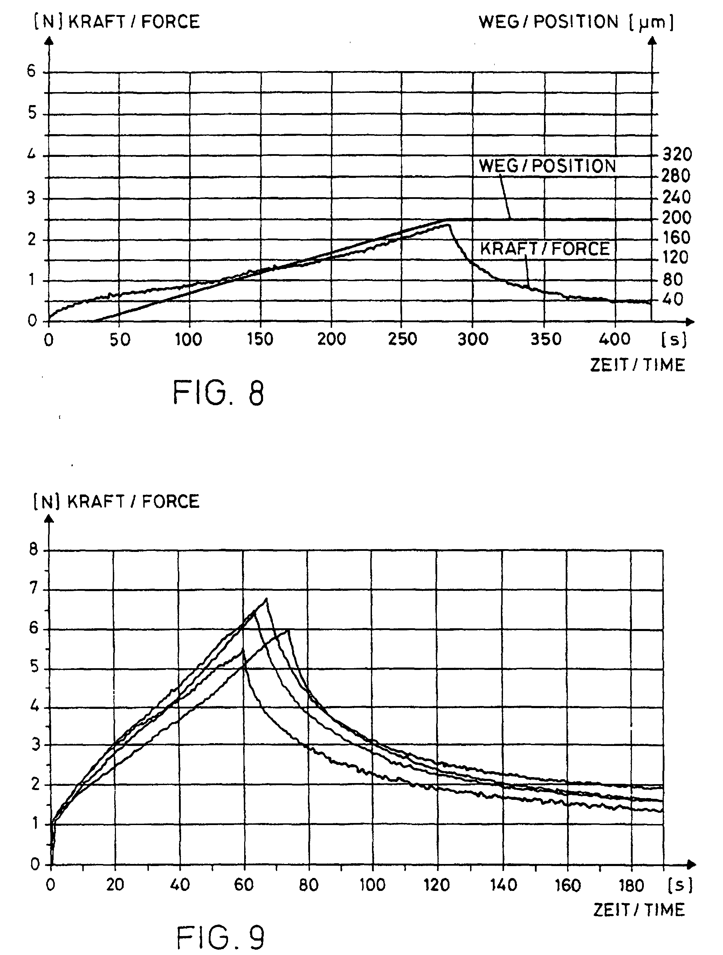

**Figuren 8 und 9** zeigen für einen mit dem erfindungsgemässen Verfahren hergestellten Knorpel (Figur 8) und für einen nativen Knorpel (Figur 9) Resultate von Belastungsversuchen zur Illustration der mechanischen Eigenschaften des erfindungsgemäss hergestellten Knorpels (Kulturbedingungen, bei denen der Knorpel hergestellt wurde, siehe weiter oben) verglichen mit mechanischen Eigenschaften von nativem Knorpel (Rind). Der Versuch besteht darin, einen Stempel mit einer konstanten Geschwindigkeit (1 Micrometer pro Sekunde) in den Knorpel zu drücken und den Stempel bei einer Eindringtiefe von 200µm anzuhalten, während dessen die Stempelkraft registriert wird. Dabei steigt die Stempelkraft zuerst etwa proportional mit der Eindringtiefe und sinkt nach dem Stillstand des Stempels ab (viskoelastischer Kraftabbau durch Flüssigkeitsverlust des Knorpelgewebes).

Die beiden Figuren 8 und 9 stellen die Stempelkraft in Newton (N) als Funktion der Zeit in Sekunden (s) dar. In der Figur 8 ist auch die Registrierung der Eindringtiefe (Weg) in µm dargestellt.

Der Belastungsversuch mit dem nach dem erfindungsgemässen Verfahren hergestellten Knorpel (Figur 8) wurde mit einem Stempel mit 20mm Durchmesser durchgeführt, sodass sich eine maximale Druckspannung von ca. 0,8N/cm² ergibt. Der Versuch mit dem nativen Knorpel wurde mit einem Stempel von 5mm Durchmesser durchgeführt, sodass sich eine maximale Druckspannung von ca. 30N/cm² ergibt.

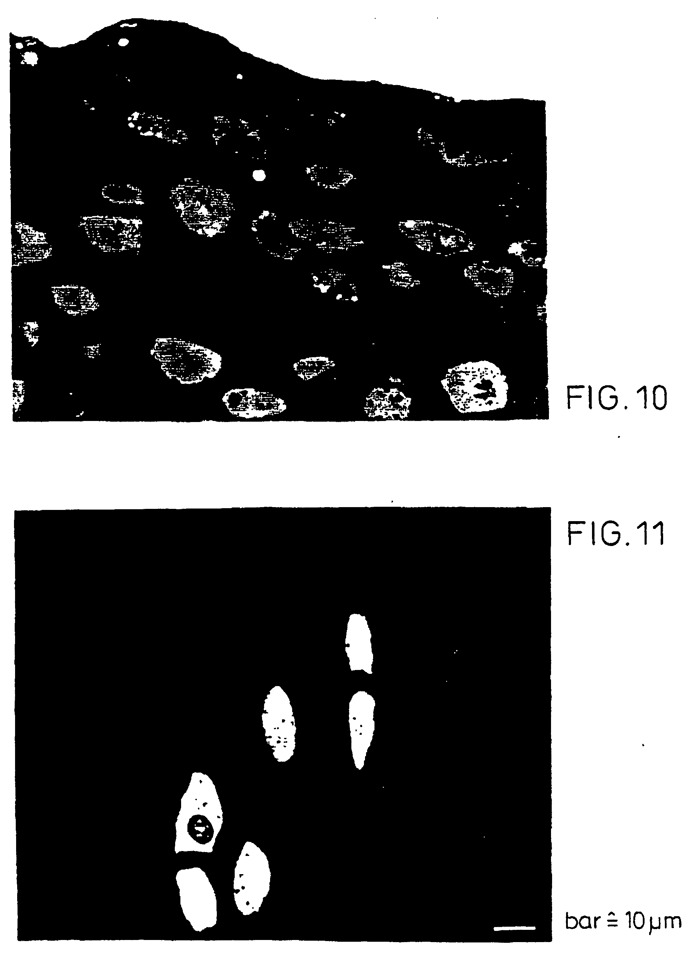

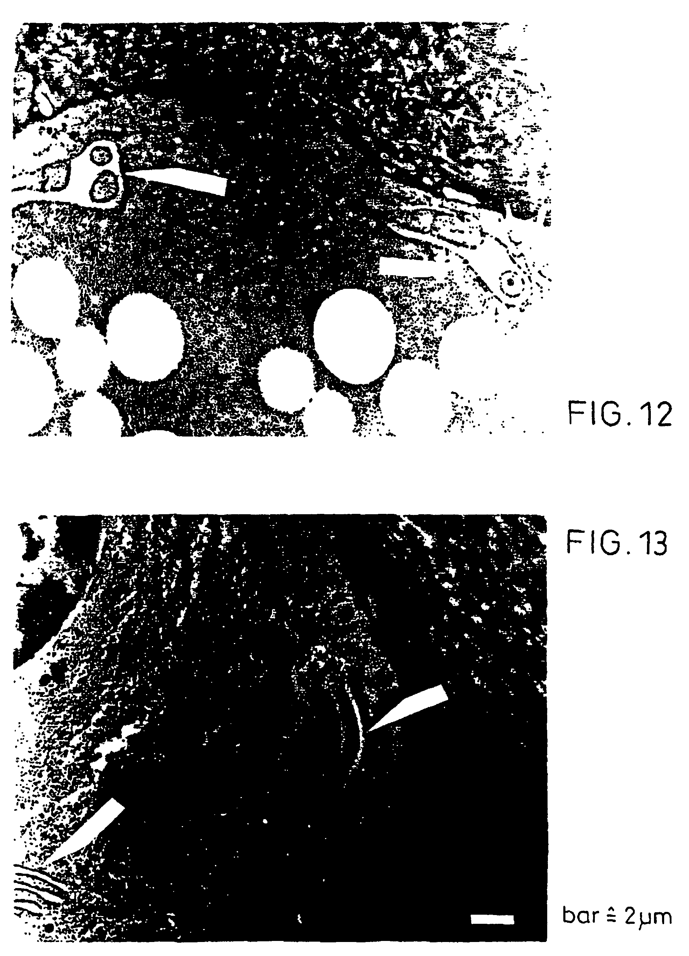

Die doch bedeutend kleinere maximale Druckspannung des Knorpels, der mit dem erfindungsgemässen Verfahren hergestellt wurde, lässt sich aus den Figuren 10 bis 13 erklären. Diese Figuren stellen lichtmikroskopische Aufnahmen (Figuren 10 und 11) und elektronenmikroskopische Aufnahmen (Figuren 12 und 13) des nach dem erfindungsgemässen Verfahren hergestellten und für die Belastungsversuche verwendeten Knorpels (Figuren 10 und 12) und menschlichen Knorpels aus der mittleren radiären Zone (Figuren 11 und 13) dar.

Die Figuren 10 und 11 zeigen deutlich, dass der nach dem erfindungsgemässen Verfahren hergestellte Knorpel bedeutend mehr Chondrozyten enthält als der Vergleichsknorpel, was für den in vitro gezüchteten Knorpel als Wachstumsstadium interpretiert werden kann. Dieselbe Interpretation legen die Figuren 12 und 13 nahe, auf denen in den Chondrozyten Organellen gut sichtbar sind (mit Pfeilen markiert). Im Vergleichsknorpel sind die Organellen schmal, was auf eine geringe Synthesetätigkeit schliessen lässt; im erfindungsgemäss hergestellten Knorpel sind sie deutlich erweitert, was auf intensive Synthesetätigkeit und damit auf eine Wachstumsphase hinweist.

Weitere elektronenmiksoskopische Untersuchungen von nach dem erfindungsgemässen Verfahren hergestelltem Knorpelgewebe zeigen, dass die Kollagenfibrillen darin ein dichtes Netz bilden, dass sie aber weniger dick sind als in nativem Knorpel (nach abgeschlossenem Wachstum) und dass sie in diesem Netz nicht gerichtet sondern beliebig angeordnet sind. Das nach dem erfindungsgemässen Verfahren hergestellte Knorpelgewebe ist also als eine Art embryonales Knorpelgewebe zu betrachten, das aber die Fähigkeit besitzt, sich in vivo (nach der Implantation) zu einem "ausgewachsenen" Knorpelgewebe weiterzuentwickeln.

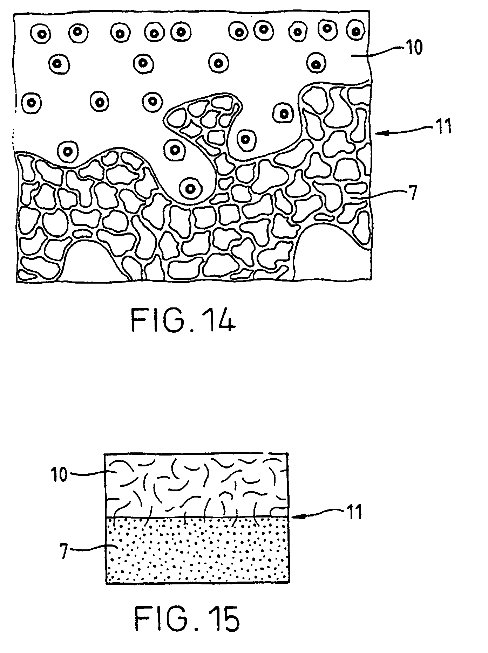

**Figur 14** zeigt in schematischer Darstellung einen histologischen Schnitt (Vergrösserung ca. 100x) durch den Grenzbereich zwischen Knorpelschicht und Knochenersatzplatte eines Implantates, das in einer Anordnung gemäss einer der Figuren 2 bis 4 hergestellt wurde. Knorpelgewebe 10 und Knochenersatzplatte 7 sind in einem Grenzbereich 11 Formschluss-artig miteinander verbunden, dadurch, dass das Knorpelgewebe in Oberflächenunebenheiten der Knochenersatzplatte 7 hineingewachsen ist.

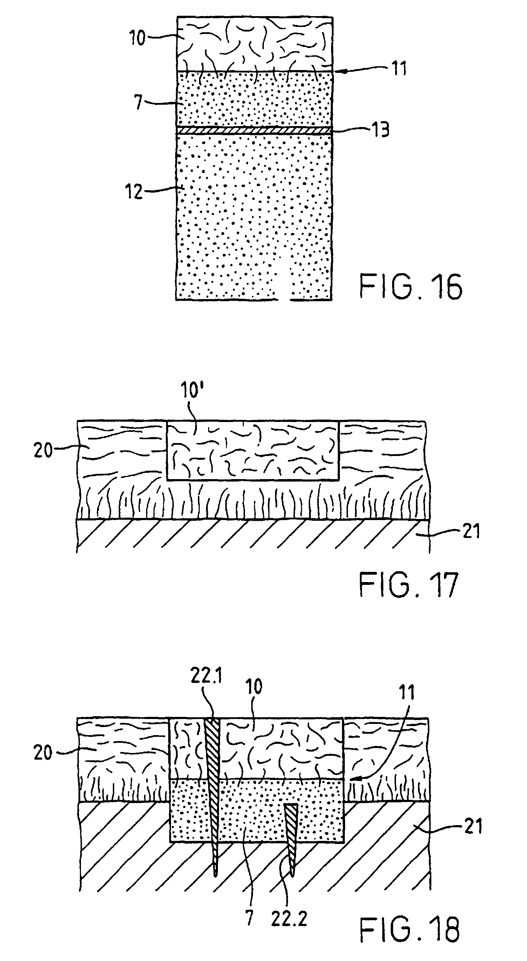

**Figuren 15 und 16** zeigen im Schnitt durch die Knorpelschicht 10 beispielhafte Ausführungsformen von Implantaten, die hergestellt werden nach Verfahren, wie sie im Zusammenhang mit den Figuren 2 bis 4 beschrieben wurden, und die zwischen der Knorpelschicht 10 und einer darunterliegenden Knochenersatzplatte 7 einen verwachsenen Grenzbereich 11 aufweisen, wie er in der Figur 14 dargestellt ist.

Nach Abschluss der Kulturzeit von einigen Wochen wird die Knochenersatzplatte 7 mit der darauf entstandenen Knorpelschicht 10 von den übrigen Wandbestandteilen des Zellraumes getrennt. Vor der Implantation wird das aus Knorpelschicht 10 und Knochenersatzplatte 7 bestehende Implantat gegebenenfalls auf eine vorgegebene Grösse und Form reduziert und/oder auf ein weiteres Stück eines Knochenersatzmaterials 12 aufgesetzt.

Für eine Bearbeitung des im Zellraum entstandenen Implantates werden in der Chirurgie gängige Methoden verwendet, wie beispielsweise Stanzen, Laser-Schneiden oder Fräsen. Für eine Vergrösserung des Knochenersatzteils wird ein weiteres Stück 12 aus einem gleichen oder anderen Knochenersatzmaterial auf der der Knorpelschicht 10 gegenüberliegenden Seite der Knochenersatzplatte 7 mit Hilfe einer Schicht 13 aus einem an sich bekannten, vorzugsweise biologisch abbaubaren Zement angesetzt.

Nach der Implantation wandern in das offenporige Knochenersatzmaterial der Knochenersatzplatte 7 und des angesetzten Stücks 12 aus der nativen Umgebung knochenbildende Zellen ein und wachsen Mikrogefässe in die Poren des Materials. Als Folge davon entsteht natürliches Knochengewebe, das das Knochenersatzmaterial (7, 12, 13), das sukzessive abgebaut wird, ersetzt. Dabei ist zu erwarten, dass der in vitro gezüchtete Knorpel im Grenzbereich 11 mineralisiert wird.

**Figuren 17 bis 20** zeigen enchondrale und osteochondrale Defekte, die mit beispielhaften Ausführungsformen von endungsgemässen Implantaten repariert sind.

**Figur 17** zeigt einen zu einer definierten Form aufgebohrten oder ausgeschnittenen, enchondralen Defekt, einen Defekt also, der in der nativen Knorpelschicht 20 liegt und das unter der Knorpelschicht 20 liegende Knochengewebe 21 nicht tangiert. Ein derartiger Defekt ist im Humanfall höchstens etwa 3mm tief und kann sich über einen beliebigen Bereich einer Knorpelschicht erstrecken. In den aufgebohrten oder ausgeschnittenen Defekt wird ein Stück Knorpelgewebe 10' eingesetzt, das beispielsweise in einer Anordnung gemäss Figur 1 gezüchtet wurde und das nach der Züchtung gegebenenfalls durch Schneiden oder Stanzen auf die Form des Defektes gebracht wurde.

Bei kleinen Defekten genügt es, für eine genügende Fixierung des Implantates im Defekt. dieses beim Einbringen leicht elastisch zu deformieren (press fit).

Bei grösseren Defekten wird das Implantat mit bekannten Mitteln befestigt: beispielsweise mit einem Stück Knochenhaut, das darüber genäht oder geklebt wird, mit einem Kleber, der zwischen nativem Knorpel und Implantat eingebracht wird (z.B. Fibrinkleber), oder durch Vernähen mit dem nativen Knorpel.

**Figur 18** zeigt einen zu einer definierten Form aufgebohrten, kleineren osteochondralen Defekt (flächige Ausdehnung bis ca. 10mm, Tiefe bis zu ca 3mm im Knochen), ein Defekt also, der nicht nur die native Knorpelschicht 20 sondern auch das darunterliegende Knochengewebe 21 betrifft. Dieser Defekt ist mit einem Implantat gemäss Figur 15 repariert, welches Implantat mit einem oder mehreren Pins befestigt ist. Es sind zwei Pins dargestellt. Der eine Pin 22.1 wird vom Chirurgen von der Oberfläche durch das Implantat getrieben, der andere Pin 22.2 ist vorgängig in der Knochenersatzplatte 7 des Implantates angeordnet worden und wird durch Pressen auf die Implantatsoberfläche in den Knochen getrieben. Je nach flächiger Ausdehnung des Defektes werden ein Pin oder eine Mehrzahl von Pins angeordnet.

Selbstverständlich ist es auch möglich, das Implantat gemäss Figur 18 mit anderen Fixierungsmitteln als mit Pins im Defekt zu befestigen.

Aus der Figur 18 ist ersichtlich, dass die beträchtlichen Scherkräfte, die bei der Belastung von Gelenken auf den Grenzbereich zwischen Knochen 21 und Knorpel 20 wirken, im Bereiche des reparierten Defektes vom Grenzbereich 11 übernommen werden, in dem die in vitro gezüchtete Knorpelschicht 10 mit der Knochenersatzplatte 7 verwachsen ist.

Anstelle des aus einer in vitro gezüchteten Knorpelschicht 10 und einer Knochenersatzplatte 7 bestehenden Implantates, wie es in der Figur 18 dargestellt ist, kann derselbe Defekt auch mit einer Füllmasse gefüllt und mit einem in vitro gezüchteten Stück Knorpelgewebe, wie es in der Figur 17 dargestellt ist, repariert werden. Zur Aufnahme der Scherkräfte sind in einem solchen Falle beispielsweise durchgehende Pins 22.1 vorzusehen.

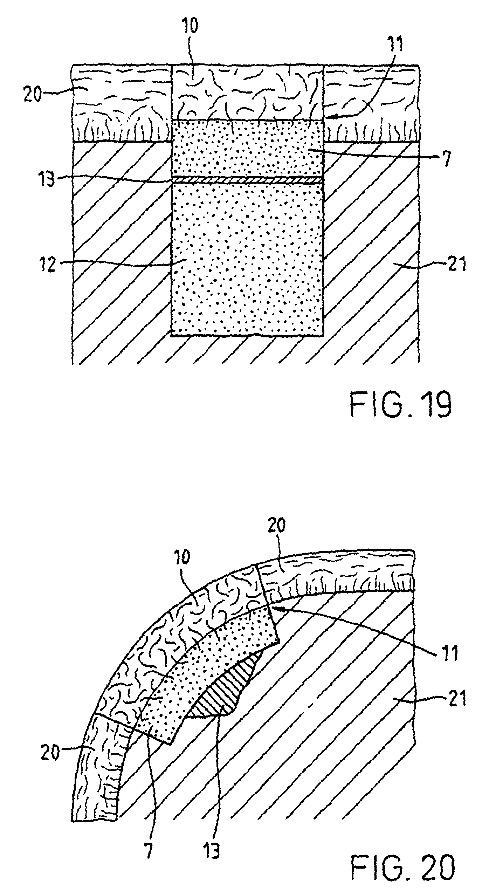

**Figur 19** zeigt einen aufgebohrten, osteochondralen Defekt, der bis in eine Tiefe von beispielsweise 20 bis 30mm präpariert und durch Implantation eines erfindungsgemässen Implantates gemäss Figur 16 repariert worden ist. Scherkräfte auf das Implantat werden wiederum durch die Verwachsung zwischen Knorpelschicht 10 und Knochenersatzplatte 7 aufgenommen. Da die Zementverbindung 13 im nativen Knochen 21 liegt, wird sie auf Scherung nicht belastet und braucht aus diesem Grunde nicht durch einen Stift verstärkt zu werden.

Die in der Figur 18 dargestellte Reparaturstelle kann auch erstellt werden, indem der untere Teil der Bohrung mit einem Knochenersatzmaterial gefüllt wird und indem auf diesem Material gegebenenfalls mittels einer Zementschicht 13 ein Implantat gemäss Figur 15 implantiert wird.

Für grössere osteochondrale Defekte können im Sinne einer Mosaikplastik eine Mehrzahl von Implantaten gemäss Figur 19 vorgesehen werden.

Figur 20 zeigt einen grossen und tiefen osteochondralen Defekt. Dieser ist derart gross, dass die neu zu erstellende Knorpelfläche nicht mehr mit einer ebenen Fläche angenähert werden kann. Ein derartiger Defekt kann, wie weiter oben angedeutet, durch eine Mosaikplastik repariert werden. Da aber die erfindungsgemässen Implantate in ihrer flächigen Ausdehnung nicht beschränkt sind und da deren Knochenersatzplatten 7 auch aus einem plastisch verformbaren Material bestehen können, lässt sich der Defekt einfacher gemäss Figur 20 mit einem Implantat gemäss Figur 15 reparieren. Zu diesem Zwecke wird der Defekt auf eine Tiefe von einigen Millimetern in den Knochen 21 zu einer definierten Form ausgeschnitten, werden tiefere Stellen mit einem plastischen Knochenersatzmaterial gefüllt und wird das Implantat im Defekt positioniert und mit geeigneten Mitteln fixiert.

## Patentansprüche

1. Verfahren zur in vitro Herstellung von Knorpelgewebe und von Implantaten, die mindestens teilweise aus Knorpelgewebe bestehen, ausgehend von vitalen Zellen, die die Fähigkeit haben, eine extrazelluläre Knorpelmatrix zu bilden, **dadurch gekennzeichnet, dass** die Zellen in einen Zellraum (1) eingebracht und zur Bildung einer extrazellulären Knorpelmatrix in diesem Zellraum (1) belassen werden, wobei keine Haftfäktoren notwendig sind, wobei die Zellen mindestens anfänglich nicht immobilisiert sind, wobei pro cm³ Zellraum 5x10⁷ bis 10⁹ Zellen eingebracht werden und wobei der Zellraum (1) mindestens teilweise durch eine semipermeable Wandung oder eine als Konvektionsbarriere wirksame offenporige Wandung von einem den Zellraum (1) umgebenden Kulturmedium-Raum (2) getrennt ist.

2. Verfahren nach Anspruch 1, **dadurch gekennzeichnet, dass** aus entsprechenden Geweben isolierte Chondrozyten, mesenchymale Stammzellen, andere mesenchymale Zellen oder Fibroblasten oder Mischungen der genannten Zelltypen oder Gewebeteilchen, die mindestens einen Typ der genannten Zellen enthalten, in den Zellraum (1) eingebracht werden.

3. Verfahren nach Anspruch 2, **dadurch gekennzeichnet, dass** die Zellen vor dem Einbringen in den Zellraum (1) in einer Zellkultur vermehrt werden.

4. Verfahren nach einem der Ansprüche 1 bis 3, **dadurch gekennzeichnet, dass** der Zellraum (1) während der Kulturzeit im Kulturmedium-Raum (2) stationär angeordnet ist oder darin bewegt wird und dass mindestens ein Teil der Zellraumwandung aus einer semipermeablen Membran mit einer Durchlässigkeit von 10'000 bis 100'000 Dalton besteht.

5. Verfahren nach Anspruch 4, **dadurch gekennzeichnet, dass** innerhalb des Zellraumes eine offenporige, starre oder plastisch deformierbare Platte (7) aus einem Knochenersatzmateriat angeordnet ist.

6. Verfahren nach einem der Ansprüche 1 bis 3, **dadurch gekennzeichnet, dass** der Zeitraum (1) während der Kulturzeit im Kulturmedium-Raum (2) stationär angeordnet ist und dass mindestens ein Teil der Zellraumwandung als offenporige Schicht mit Poren von bis zu ca. 20µm ausgebildet ist

7. Verfahren nach Anspruch 6, **dadurch gekennzeichnet, dass** die offenporige Schicht eine Platte (7) aus einem Knochenersatzmaterial ist und dass der Zellraum (1) derart angeordnet ist, dass die Platte (7) unten ist, sodass die in den Zellraum (1) eingebrachten Zellen sich auf der Platte (7) absetzen.

8. Anordnung zur Durchführung des Verfahrens nach einem der Ansprüche 1 bis 7, **dadurch gekennzeichnet, dass** sie einen Zellraum (1) aufweist, der eine flächige Form mit zwei im wesentlichen parallelen Stirnflächen hat und dessen zwei Stirnflächen durch semipermeable Membrane (3) oder durch als Konvektionsbarriere dienende, offenporige Schichten gebildet sind, wobei der Zellraum (1) in einem ihn umgebenden Kulturmedium-Raum (2) angeordnet ist, mit dem er durch die semipermeablen Membrane (3) oder die offenporigen Schichten in Verbindung steht

9. Anordnung nach Anspruch 8, **dadurch gekennzeichnet, dass** die semipermeable Membran (3) eine Durchlässigkeit von 10'000 bis 100'000 Dalton hat.

10. Anordnung nach Anspruch 8, **dadurch gekennzeichnet, dass** die als Konvektionsbarriere dienende Schicht eine offene Porosität von 1 bis 20µm aufweist und eine Dicke von 0,5 bis 3mm hat.

11. Anordnung nach Anspruch 10, **dadurch gekennzeichnet, dass** die offenporige Schicht eine starre oder plastisch verformbare Platte (7) aus einem biologisch abbaubaren oder nicht abbaubaren Knochenersatzmaterial ist und dass der Zellraum im Kulturmedium-Raum (2) derart angeordnet ist, dass die Knochenersatzplatte (7) unten ist.

12. Anordnung nach Anspruch 11, **dadurch gekennzeichnet, dass** die Knochenersatzplatte (7) auf ihrer gegen das Innere des Zellraumes gewandten Oberfläche Unebenheiten mit Grössen von mindestens 20µm aufweist.

13. Anordnung nach einem der Ansprüche 11 oder 12, **dadurch gekennzeichnet, dass** die Knochenersatzplatte (7) auf ihrer vom Zellraum abgewandten Seite eine Porosität von 300 bis 700µm aufweist.

14. Anordnung nach einnem der Ansprüche 11 bis 13, **dadurch gekennzeichnet, dass** ausserhalb der Knochenersatzplatte (7) eine weitere durchlässige Wandung angeordnet ist.

15. Zellraum begrenzt durch eine Wandung, die mindestens zum Teil aus einem offenporigen Knochenersatzmateflal besteht, als Teil einer Anordnung nach einem der Ansprüche 12 bis 14.

16. Implantat hergestellt nach dem Verfahren gemäss einem der Ansprüche 5 bis 7, **dadurch gekennzeichnet, dass** es eine Platte (7) aus einem offenporigen Knochenersatzmaterial aufweist, deren Oberfläche mindestens teilweise mit einer in vitro gezüchteten Knorpelschicht (10) bedeckt ist, wobei das Knorpelgewebe der Knorpelschicht (10) durch Einwachsen in Poren und Oberflächenunebenheiten des Knochenersatzmaterials in diesem verankert ist.

17. Implantat nach Anpruch 16, **dadurch gekennzeichnet, dass** die Platte (7) aus Knochenersatzmaterial mindestens in dem der Knorpelschicht (10) zugewandten Bereich Poren in der Grösse von 1 bis 20µm aufweist und eine Dicke von 0,5 bis 3mm hat.

18. Implantat nach einem der Ansprüche 16 oder 17, **dadurch gekennzeichnet, dass** die Platte (7) aus Knochenersatzmaterial durch eine Zementschicht (13) mit einem weiteren Teil (12) aus Knochenersatzmaterial verbunden ist.

19. Implantat nach einem der Ansprüche 16 bis 18, **dadurch gekennzeichnet, dass** es starr oder plastisch verformbar ist.

20. Verwendung des Verfahrens nach einem der Ansprüche 1 bis 7 zur Herstellung von Implantaten zur Reparatur von enchondralen oder osteochondralen Gelenk-Defekten.

21. Verwendung des Verfahrens nach einem der Ansprüche 1 bis 4 oder 6 zur Herstellung von Gehörknochen, Nasenknorpeln, Orbitalböden, Ohrenmuscheln oder von Teilen davon.

## Claims

1. Method for in vitro production of cartilage tissue and of implants which consist at least partly of cartilage tissue, starting from vital cells which have the ability to produce an extracellular cartilage matrix, **characterized in that** the cells are brought into a cell-space (1) and are left in this cell space (1) for forming an extracellular cartilage matrix, wherein no adhesion factors are necessary, wherein the cells are at least preliminarily not immobilized, wherein per cm³ of the cell space a density of 5x10⁷ to 10⁹ cells is introduced and wherein the cell space (1) is at least partly separated from a surrounding culture medium space (2) by a semi-permeable wall or by an open-pore wall acting as convection barrier.

2. Method according to claim 1, **characterized in that** the cells brought into the cell space (1) are chondrocytes, mesenchymal stem cells, fibroblasts or mixtures of said cells, or **in that** tissue particles containing at least one type of said cells are brought into the cell space (1).

3. Method according to claim 2, **characterized in that** the cells are multiplied in a cell culture before being brought into the cell space (1).

4. Method according to one of claims 1 to 3, **characterized in that** during the culture period the cell space (1) is arranged to be stationary in the culture medium space (2) or is moved in it and that at least part of the cell space wall consists of a semi-permeable membrane with a permeability of 10.000 to 100.000 Dalton.

5. Method according to claim 4, **characterized in that** an open-pore, rigid or plastically deformable plate (7) made of a bone substitute material is arranged within the cell space.

6. Method according to one of claims 1 to 3, **characterized in that** the cell space (1) is arranged to be stationary in the culture medium space (2) during the culture period and that at least part of the cell space wall is designed as an open-pore layer with pores of a size of ca. 20µm.

7. Method according to claim 6, **characterized in that** the open-pore layer is a plate (7) made of a bone substitute material and that the cell space (1) is arranged such that the plate (7) is positioned such that the cells brought into the cell space (1) settle on the plate (7).

8. Arrangement for carrying out the method according to one of claims 1 to 7, **characterized in that** it comprises a cell space (1) which is of flat shape with two substantially parallel faces and these two faces are formed by semi-permeable membranes (3) or open-pore layers acting as convection barriers, wherein the cell space (1) is arranged in a surrounding culture media space (2) with which it is in connection through a semi-permeable membrane (3) or an open-pore layer acting as convection barrier.

9. Arrangement according to claim 8, **characterized in that** the semi-permeable membrane (3) has a permeability of 10.000 to 100.000 Dalton.

10. Arrangement according to claim 8, **characterized in that** the layer acting as convection barrier has an open porosity of 1 to 20µm and a thickness of 0.5 to 3mm.

11. Arrangement according to claim 10, **characterized in that** the open-pore layer is a rigid or plastically deformable plate (7) made of a biologically degradable or not degradable bone substitute material and that the cell space in the culture medium space (2) is arranged such that the bone substitute plate (7) forms the bottom of the cell space.

12. Arrangement according to claim 11, **characterized in that** the bone substitute plate (7) has a surface with a roughness in the range of at least 20µm facing the inside of the cell space.

13. Arrangement according to one of claims 11 or 12, **characterized in that** the bone substitute plate (7) has a porosity of 300 to 700µm on its side facing away from the cell space.

14. Arrangement according to one of claims 11 to 13, **characterized in that** the cell space comprises a further permeable wall outside of the bone substitute plate (7).

15. Cell space defined by a wall which at least partly consists of an open-pore bone substitute material the cell space being a part of an arrangement according to one of claims 12 to 14.

16. Implant produced with a method according to one of claims 5 to 7, **characterized in that** it comprises a plate (7) made of an open-pore bone substitute material the surface of which is at least partly covered with a cartilage layer (10) cultivated in vitro, whereby the cartilage tissue of the cartilage layer (10) is anchored in the bone substitute material by growing into the pores and surface unevenesses of this material.

17. Implant according to claim 16, **characterized in that** the plate (7) made of bone substitute material comprises, at least in the region facing the cartilage layer (10), pores of a size of 1 to 20µm and has a thickness of 0.5 to 3mm.

18. Implant according to one of claims 16 or 17, **characterized in that** the plate (7) made of bone substitute material is connected to a further part (12) made of bone substitute material by means of a cement layer (13).

19. Implant according to one of claims 16 to 18, **characterized in that** it is rigid or plastically deformable.

20. Use of the method according to one of claims 1 to 7 for producing implants for repairing enchondral or osteochondral joint defects.

21. Use of the method according to one of claims 1 to 4 or 6 for producing auditory bones, nose cartilage, orbital floors, ear conchs or parts thereof.

## Revendications

1. Procédé pour la production in vitro de cartilage et d'implants qui sont constitués au moins en partie de cartilage, à partir de cellules vitales qui ont la faculté de former une matrice de cartilage extra-cellulaire, **caractérisé en ce que** les cellules sont introduites dans un compartiment de cellules (1) et laissées dans ce compartiment (1) pour former une matrice de cartilage extra-cellulaire, aucun facteur de cohésion n'étant nécessaire, les cellules n'étant pas immobilisées, tout au moins au début, 5x10⁷ à 10⁹ de cellules étant introduites par cm³ de compartiment de cellules et le compartiment de cellules (1) étant séparé d'un compartiment de milieu de culture (2) entourant le compartiment de cellules (1) au moins en partie par une paroi semi-perméable ou une paroi à pores ouverts agissant comme une barrière de convection.

2. Procédé selon la revendication 1, **caractérisé en ce que** des chondrocytes isolés de tissus correspondants, des cellules souches mésenchymales, d'autres cellules mésenchymales ou des fibroplastes ou des mélanges des types de cellules citées ou des particules de tissu qui contiennent au moins un type des cellules citées, sont introduits dans le compartiment de cellules (1).

3. Procédé selon la revendication 2, **caractérisé en ce que** les cellules se multiplient dans une culture cellulaire avant leur introduction dans le compartiment de cellules (1).

4. Procédé selon l'une quelconque des revendications 1 à 3, **caractérisé en ce que** le compartiment de cellules (1), pendant le temps de culture, est disposé de façon stationnaire dans le compartiment du milieu de culture (2) ou s'y déplace et qu'au moins une partie de la paroi du compartiment de cellules est constituée d'une membrane semi-perméable avec une perméabilité de 10 000 à 100 000 daltons.

5. Procédé selon la revendication 4, **caractérisé en ce qu'**une plaque à pores ouverts, rigide ou déformable plastiquement (7), en succédané d'os, est disposée à l'intérieur du compartiment de cellules.

6. Procédé selon l'une quelconque des revendications 1 à 3, **caractérisé en ce que** le compartiment de cellules (1) est disposé, pendant le temps de culture, de manière stationnaire dans le compartiment de milieu de culture et qu'au moins une partie de la paroi du compartiment de cellules est formée comme une couche à pores ouverts avec des pores allant jusqu'à environ 20 µm.

7. Procédé selon la revendication 6, **caractérisé en ce que** la couche à pores ouverts est une plaque (7) en succédané d'os et que le compartiment de cellules (1) est disposé de telle sorte que la plaque (7) soit en bas, de façon à ce que les cellules introduites dans le compartiment de cellules (1) se déposent sur la plaque (7).

8. Agencement pour la mise en oeuvre du procédé selon l'une quelconque des revendications 1 à 7, **caractérisé en ce qu'**il présente un compartiment de cellules (1) qui a une forme plate avec deux faces frontales essentiellement parallèles et dont les deux faces frontales sont formées par des membranes semi-perméables (3) ou par des couches à pores ouverts servant de barrière de convection, le compartiment de cellules (1) étant disposé dans un compartiment de milieu de culture (2) qui l'entoure et avec lequel il est en liaison par les membranes semi-perméables (3) ou les couches à pores ouverts.

9. Agencement selon la revendication 8, **caractérisé en ce que** la membrane semi-perméable (3) a une perméabilité de 10 000 à 100 000 daltons.

10. Agencement selon la revendication 8, **caractérisé en ce que** la couche servant de barrière de convection présente une porosité ouverte de 1 à 20 µm et a une épaisseur de 0,5 à 3 mm.

11. Agencement selon la revendication 10, **caractérisé en ce que** la couche à pores ouverts est une plaque rigide ou déformable plastiquement (7), réalisée dans un succédané d'os biologiquement dégradable ou non, et que le compartiment de cellules est disposé dans le compartiment de milieu de culture (2) de telle façon que la plaque en succédané d'os (7) soit en bas.

12. Agencement selon la revendication 11, **caractérisé en ce que** la plaque en succédané d'os (7) présente, sur sa surface tournée vers l'intérieur du compartiment de cellules, des inégalités d'une dimension d'au moins 20 µm.

13. Agencement selon l'une des revendications 11 ou 12, **caractérisé en ce que** la plaque en succédané d'os (7) présente, sur sa face opposée au compartiment de cellules, une porosité de 300 à 700 µm.

14. Agencement selon l'une quelconque des revendications 11 à 13, **caractérisé en ce qu'**une autre paroi perméable est disposée à l'extérieur de la plaque en succédané d'os (7).

15. Compartiment de cellules limité par une paroi qui est constituée au moins en partie d'un succédané d'os à pores ouverts en tant que partie d'un agencement selon l'une des revendications 12 à 14.

16. Implant produit suivant le procédé selon l'une des revendications 5 à 7, **caractérisé en ce qu'**il présente une plaque (7) en succédané d'os dont la surface est recouverte au moins en partie avec une couche de cartilage cultivé in vitro, le cartilage de la couche de cartilage (10) étant ancré dans le succédané d'os par croissance dans les pores et les inégalités de surface de celui-ci.

17. Implant selon la revendication 16, **caractérisé en ce que** la plaque (7) en succédané d'os présente, au moins dans la zone tournée vers la couche de cartilage (10), des pores d'une grosseur de 1 à 20 µm et a une épaisseur de 0,5 à 3 mm.

18. Implant selon l'une des revendications 16 ou 17, **caractérisé en ce que** la plaque (7) en succédané d'os est reliée par une couche de ciment (13) avec une autre partie (12) en succédané d'os.

19. Implant selon l'une quelconque des revendications 16 à 18, **caractérisé en ce qu'**il est rigide ou déformable plastiquement.

20. Utilisation du procédé selon l'une quelconque des revendications 1 à 7 pour la production d'implants pour la réparation d'anomalies enchondrales ou ostéo-chondrales des articulations.

21. Utilisation du procédé selon l'une quelconque des revendications 1 à 4 ou 6, pour la production de chaînes d'osselets, de cartilage du nez, de fonds d'orbites, de pavillons d'oreilles ou de parties de ceux-ci.
